# FASCICULE DE BREVET EUROPEEN

(11) **EP 1 112 079 B1**
(45) Date de publication et mention de la délivrance du brevet: **17.04.2002**
(21) Numéro de dépôt: 99942972.3
(22) Date de dépôt: 15.09.1999
(51) Int. Cl.: A61K 35/78, A61P 17/06, A61P 17/10, A61P 17/12, A61P 17/00

(54) **EXTRAIT DE MYRTE, PROCEDE DE PREPARATION ET APPLICATION**
MYRTEEXTRAKT, VERFAHREN ZUR HERSTELLUNG UND VERWENDUNG
MYRTLE EXTRACT, PREPARATION METHOD AND USE

(30) Priorité: 17.09.1998 FR 9811619
(43) Date de publication de la demande: 04.07.2001
(73) Titulaire: Pierre Fabre Dermo-Cosmetique, 92100 Boulogne-Billancourt (FR)
(72) Inventeur: PUYBARET, Christel, F-31240 L'Union (FR); DAVID, Bruno, F-31650 Saint-Orens de Gameville (FR); CHARVERON, Marie, F-31000 Toulouse (FR); MAMATAS, Stylianos, Rés. les Cyclades, F-31400 Toulouse (FR)
(74) Mandataire: Ahner, Francis
(86) Numéro de dépôt international: FR9902189
(87) Numéro de publication internationale: WO0016791

(56) Documents cités:
- FR-A- 2 735 026
- FR-A- 2 741 265
- A.ROTSTEIN ET AL.: "ISOLATION AND ANTIBACTERIAL ACTIVITY OF ACYLPHLOROGLUCINOLS FROM MYRTUS COMMUNIS." ANTIMICROBIAL AGENTS AND CHEMOTHERAPY, vol. 6, no. 5, novembre 1974 (1974-11), pages 539-542, XP002105891

## Description

La présente invention concerne un extrait de parties aériennes de Myrte (*Myrtus communis L.,* Myrtaceae) et un procédé de préparation de cet extrait. Cet extrait présente un intérêt dans le traitement des syndromes inflammatoires cutanés et des syndromes de différentiation cutané comme notamment le psoriasis et les troubles de la kératinisation. La présente invention concerne encore une composition dermatologique et/ou cosmétique et l'utilisation de cette composition en particulier pour le traitement du psoriasis. Diverses formes galéniques illustrent l'invention sans en limiter la portée.

La présente invention concerne plus particulièrement un extrait de parties aériennes de Myrte, cet extrait est une fraction apolaire qui contient des composés acylphloroglucinols, en outre cet extrait est essentiellement exempt de chlorophylles. Parmi les composés acylphloroglucinols contenus dans cet extrait, on compte des myrtucommulones et en particulier la myrtucommulone-B'. Des triterpènes et des stérols sont également présents. Le titre de myrtucommulone-B' est compris entre 2% et 12 %, de préférence compris entre 4% et 8% et de manière encore plus préférée entre 5% et 7% et le titre en "triterpènes et stérols" est compris entre 30 % et 50 % et de préférence compris entre 37 % et 43 %.

Au sens de la présente description, le titre est défini comme la masse de la substance considérée (substance titrée) par rapport à la masse du produit final (volume de l'extrait de Myrte).

La présente invention concerne aussi un procédé de préparation d'un extrait de parties aériennes de Myrte tel qu'il comprend une étape dans laquelle les parties aériennes de Myrte sont soumises à une extraction au moyen d'un solvant, une étape d'élimination des chlorophylles de préférence effectuée au moyen de charbon actif et une étape dans laquelle les composés apolaires sont isolés, cette étape est par exemple une extraction liquide/liquide, une précipitation en évaporant le solvant organique sur l'eau. Le solvant le plus souvent utilisé pour effectuer l'extraction sur les parties aériennes de la plante est un alcool de préférence choisi dans le groupe formé par l'éthanol, le méthanol, l'isopropanol, ou une cétone de préférence choisie dans le groupe formé par l'acétone, la méthyléthylacétone. On peut cependant aussi employer l'hexane, le chlorure de méthylène, l'éther isopropylique, l'acétate d'éthyle.

L'extrait selon la présente invention peut être obtenu par le procédé selon la présente invention.

La présente invention concerne aussi une composition dermatologique et/ou cosmétique qui comprend un extrait selon la présente invention et l'utilisation de ladite composition pour le traitement du psoriasis, des troubles de la kératinisation de la peau et du cuir chevelu, les parakératoses, la dermatite atopique, les xéroses, les diskératoses, les ichtyoses, l'acné.

Le psoriasis est une maladie difficile à traiter qui affecte environ 2 à 3 % de la population mondiale. Cette affection, sans modifier la durée de vie des patients, en altère considérablement la qualité. Les thérapeutiques disponibles à l'heure actuelle sont ciblées sur les facteurs d'amplification responsables de la prolifération et de l'inflammation psoriasique.

Le psoriasis, un désordre inflammatoire cutané, est caractérisé par une hyperprolifération accentuée des kératynocytes associée à une vascularisation significativement accrue de la peau, une activation des fibroblastes, une infiltration des fibroblastes, une infiltration de leucocytes, des modifications du métabolisme des eicosanoïdes et de la production des cytokines.

Sa définition repose sur des critères cliniques histologiques et évolutifs. Cliniquement, il est caractérisé par la présence de plaques érythémateuses bien limitées, recouvertes de squames épaisses blanchâtres. Le nombre et la localisation des lésions sont variables. Le plus fréquemment, le psoriasis siège sur des régions exposées aux contacts extérieurs et symétriques (coude, genou). D'un point de vue histologique, les squames présentent un aspect hyperkératosique et parakératosique (persistance anormale des noyaux dans les couches superficielles). Elles correspondent à un épaississement très important de l'épiderme avec une kératinisation anormale. Le derme est le siège d'une inflammation plus ou moins intense.

La peau est un organe en contact permanent avec d'une part l'extérieur, l'environnement, et d'autre part, avec les tissus profonds de notre organisme. De ce fait, l'intégrité de cette structure cutanée joue un rôle primordial dans les communications cellules-cellules, et de là avec la différenciation des divers tissus. La peau est aussi un organe unique pouvant être reconstruit in vitro et représentant ainsi un outil d'étude de la prolifération et différenciation cellulaire cutanée.

Au niveau de l'épiderme humain, il existe deux populations de kératinocytes : les cellules souches avec un haut potentiel de prolifération et les cellules avec un potentiel de prolifération transitoire, qui sont responsables de la différenciation terminale du tissu. Durant leur évolution, de la couche basale jusqu'à la desquamation, les kératinocytes subissent de grands changements biochimiques et morphologiques. Les cellules des couches basale et suprabasale synthétisent des cytokératines qui s'organisent en filaments et faisceaux de filaments (NELSON W. et al., 1983 : "The 50 and 58 Kda keratin classes as molecular markers for stratified squamous epithelia : cell culture studies", J. Cell. Biol., 1997, 244-51) ; (EICHNER R. et al, 1996 : "The role of keratin subfamilies and keratin pairs in the formation of human epidermal intermediate
filaments", J. Cell. Biol., 102, 1767-77). Les cellules de la couche granuleuse produisent d'autres protéines marqueurs de différenciation différentes des cytokératines K 1-K 10 telles que involucrine, loricrine, transglutaminase impliquées dans la phase terminale de formation de la couche cornée (FUCHS E., 1990 : "Epidermal differentiation : the bare essentials", J. Cell. Biol, 111, 280714) où les cellules sont métaboliquement inactives.

Récemment, des études ont mis en évidence le rôle et la distribution des glycoprotéines desmosomiales (desmogléïne) dans la morphogénèse épithéliale (ALLEN E. et al. : "Mice expressing a mutant desmosomal Cadherin Exhibit Abnormalities in Desmosomes, Proliferation and Epidermal differentiation", J. Cell. Biol., 1996, 133(6), 1367-82). Ainsi, des modifications des desmosomes épidermiques chez la souris ont entraîné une augmentation de prolifération kératinocytaire et une hyperplasie épidermique. D'où la nécessité d'avoir une bonne distribution des desmosomes et des protéines desmosomiales telle la desmogléine pour la constitution d'un épiderme normal. En outre, ces glycoprotéines sont en étroite relation avec les filaments intermédiaires donc, les kératines, éléments essentiels de l'architecture épidermique.

Les kératinocytes in vitro en culture sont incapables d'assurer une différenciation terminale spontanée (ASSALINEAU D. et al., 1986 : "Human epidermis reconstructed by culture : is it normal?", J. Invest. Dermatol., 86, 181-85. Cependant, l'addition de certaines substances dans le milieu de culture, telles le calcium, les rétinoides, certains facteurs de croissance, induit un programme de différenciation terminale dépendant de la substance appliquée (FUCHS E., 1990 : "Epidermal differentiation : the bare essentials", J. Cell. Biol, 111, 2807-14). La vitamine A et les rétinoïdes sont indéniablement des régulateurs physiologiques de la différenciation épidermique terminale (DARMON M., 1991: "Retinoic acid in skin and epithelia", Semiss. Dev. Biol., 2, 219-28)(WATT F., 1989 : "Terminal diffrenciation of epidermal keratinocytes", Curr. Opin. Cell. Biol., 1, 1107-15). Un déficit ou un excès de vitamine A induit des pathologies cutanées. D'autre part, les rétinoïdes sont utilisés par voie topique ou systémique dans le traitement de pathologies dermatologiques sévères. Cependant, l'épiderme répond différemment aux rétinoïdes selon que l'on s'adresse à un traitement in vivo ou in vitro en culture de tissu.

Dans les essais in vitro, les rétinoïdes telle la vitamine A acide à concentration supra-physiologique 10⁻⁷ M induisent des modifications des cytokératines K1, K10 et des précurseurs des enveloppes cornées telle que l'involucrine.

Le psoriasis apparaît à tout âge, avec une évolution par poussées successives. Actuellement, en l'absence de traitement étiologique, le traitement local est indispensable et essentiel.

Les traitements locaux classiques comme les dermocorticoldes, les goudrons, l'anthraline, les rétinoïdes, présentent un grand nombre d'inconvénients et d'effets secondaires, et ne possèdent pas la double activité sur le versant inflammatoire et le versant prolifération.

Par exemple, les dermocorticoïdes sont responsables de vergetures extensives, d'atrophies cutanées, de dépigmentation et d'effet iatrogène systémique classique, comme l'hypercorticisme.

La plante à la base de l'invention, le Myrte commun, *Myrtus communis L.,* est un arbuste de la famille des Myrtaceae de 2 à 3 mètres de haut qui présente des feuilles opposées, coriaces, persistantes avec un pétiole court et un limbe ovalaire acuminé. Les fleurs blanches, longuement pélonculées, disposées à l'aisselle des feuilles, sont odorantes et comportent de nombreuses étamines. L'ovaire triloculaire est surmonté d'un style à stigmate unique. Le fruit est une baie charnue ovoïde de la taille d'un pois, vert puis noir-bleuâtre à maturité. Il contient de très nombreuses graines jaunâtres dépourvues d'albumen. Toutes les parties de la plante contiennent des poches schizogènes à huile essentielle qui sont responsables de la forte odeur aromatique après contusion.

Le Myrte jouit depuis l'Antiquité d'une renommée thérapeutique essentiellement basée sur la présence d'huile essentielle. Le nom latin Myrtus vient du grec Myrtos qui dérive de Myron (parfum). Les anciens l'utilisaient comme antiseptique, désinfectant, parasiticide en usage externe et même interne, notamment comme stimulant et antibronchitique. L'eau distillée des feuilles et des fleurs a eu un certain succès sous le nom d' "Eau d'Ange" jusqu'au XIXe siècle comme aromatique et astringent. Les feuilles ont été utilisées dans le traitement des plaies, des hémorroïdes et des affections pulmonaires. Autrefois, on expliquait ces effets bénéfiques par la présence de tanins et l'action antiseptique par l'activité de l'huile essentielle.

L'ouvrage de F.J. CAZIN "Traité Pratique et Raisonné des Plantes Médicinales Indigènes et Acclimatées" publié par Asselin & Houzeau à Paris en 1886 mentionne l'intérêt du "Myrte" dans le traitement du Psoriasis. "Cette plante, d'une odeur forte, aromatique, d'une saveur amère, passe pour tonique, excitante, vermifuge et antipsorique". En fait , il s'agit de *Myrica gale L.*, plante d'une famille (Myricaceae) différente du vrai Myrte (Myrtaceae), dont le nom vernaculaire est Myrte Batard. La partie de plante n'est nullement précisée, le caractère aromatique semble être important.

G. GARNIER et co-auteurs reprennent par erreur les informations de CAZIN pour le vrai Myrte (*Myrtus communis)* dans l'ouvrage "Ressources Médicinales de la Flore Française" édité par Vigot Frères à Paris en 1961. "L'huile essentielle, administrée autrefois sous le nom impropre de myrtol, douée de propriétés antiseptiques et désinfectantes, agit comme stimulant de la digestion, est hémostatique et en plus exerce une action marquée sur les affections cutanées squameuses et principalement sur le psoriasis ; cependant, administrée à doses trop élévées, elle provoque chez l'homme des nausées, de la céphalée, de l'abattement...". Selon ces informations erronées à la base, il est clair que l'huile essentielle est responsable de l'activité antipsorique.

Le Myrte est cité dans de nombreux brevets:
- Le brevet FR 2 296 401 déposé le 31 décembre 1974 mentionne le Myrte mais celui-ci est inclu dans une une liste de 7 plantes et n'est pas présenté comme possédant une activité particulière. Les extraits de Myrte, Oignon, Hammamélis, Millepertuis, Algues, Levure plus de la progestérone du placenta glycériné, du sulfate neutre d'oxyquinoléine entrent dans une composition cosmétique revendiquée comme stimulante et fortifiante pour le cuir chevelu et la racine du cheveu.
- Le brevet FR 2 669 032 utilise les feuilles de Myrte parmi un choix de 16 plantes en vue d'extraire des dérivés du tocophérol.
- Le brevet FR 2 504 551 déposé le 24 avril 1981 revendique l'utilisation de préparations contenant des huiles essentielles de diverses Lamiaceae et d'autres familles botaniques dont les Myrtaceae pour traiter des affections cutanées telles que les brûlures, piqûres, gerçures ou coups.
- Le brevet EP-347 493 cite le Myrte parmi une liste de plus de 60 espèces végétales destinées à l'extraction de l'huile essentielle par un procédé particulier. Les propriétés thérapeutiques du Myrte ne sont nullement revendiquées.
- Le brevet FR 2 735 026 revendique une composition capillaire comprenant un extrait de Myrte, son procédé de préparation et son utilisation notamment pour un traitement antipelliculaire. L'extrait mentionné est un extrait polaire enrichi en polyphénols, flavonoides et tanins.
- L'association de cet extrait avec des antifongiques est revendiquée par les mêmes inventeurs dans le brevet FR 2 741 265.

Nous avons effectué l'étude phytochimique de cette plante et avons recherché l'extrait présentant la meilleure activité anti-inflammatoire et anti-prolifératrice sur le kératinocyte en culture. L'huile essentielle de Myrte obtenue par ces procédés classiques, citée depuis plusieurs siècles comme intéressante en thérapeutique, est selon nos travaux dépourvue d'intérêt antiinflammatoire et antiprolifératif.

L'extrait selon la présente invention qui présente des propriétés intéressantes notamment dans le traitement du psoriasis, est un extrait apolaire renfermant la famille des composés acylphloroglucinols dont les myrtucommulones et comprend aussi des triterpènes et stérols. Les myrtucommulones sont des structures complexes dérivant du phloroglucinol décrites en 1974 par Y. KASMAN et al., Phytochemistry, 30, 991-997. Les formules chimiques des myrtucommulones A et B sont présentées ci-dessous. Les fractions polaires riches en tanins ne sont pas intéressantes dans notre étude. 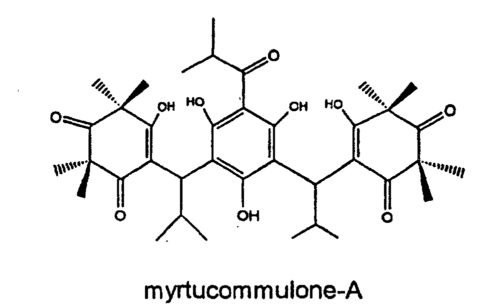 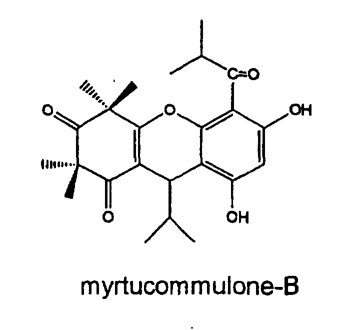 L'extrait selon la présente invention préparé à partir du Myrte, associe les avantages d'un anti-inflammatoire et d'un inhibiteur de prolifération des kératinocytes humains. L'une ou l'autre activité pharmacologique est nécessaire pour traiter efficacement le psoriasis comme nous l'avons vu dans la description de cette maladie.

En comparaison avec la vitamine A acide, molécule de référence dans le traitement du psoriasis, nous nous sommes donc proposés, dans un premier temps, de contrôler l'effet de l'extrait actif de Myrte (objet de notre invention) sur un modèle de culture de kératinocytes en milieu défini, afin d'obtenir une différenciation in vitro.

Plusieurs paramètres sont analysés:
- Analyse morphologique : contrôle de la viabilité de l'épiderme de culture : test de métabolisation du MTT [3,(4,5 diméthylthiazol) 2,5 diphenyltetrazolium bromide] en formazan par les cellules épidermiques basales et suprabasales vivantes, analyse histologique après coloration à l'Hemalum / Eosine / Safran, évaluation de la couche cornée après traitement alcalin.
- Etude des marqueurs de prolifération et différenciation cellulaire : analyse du cycle cellulaire: immunomarquage Ki67, analyse de la différenciation: immunomarquage des cytokératines K1, K10, analyse d'un précurseur de l'enveloppe cornée : immunomarquage de l'involucrine.
- Etude des marqueurs d'adhésion: marqueur d'adhésion cellule-cellule: immunomarquage de la desmogléïne 1-2.

L'étude comparative de la vitamine A acide 10⁻⁷ M, et de l'extrait actif de Myrte à diverses concentrations (0,3 ; 1 ; 3 et 5 pg/ml) nous permet de prédire l'effet de ce dernier sur l'organisation d'un épiderme en culture. Elle nous a donc permis de suivre divers marqueurs de prolifération et de différenciation cellulaire sur un modèle d'épiderme de culture. Tout comme la vitamine A acide, l'extrait de Myrte est capable de réguler le comportement prolifératif (diminution du nombre de cellules nuclées dans les couches basale et supra basale) et d'agir sur la formulation du stratum corneum en augmentant le nombre de strates. Il est capable de moduler négativement l'expression des marqueurs de différenciation K1-K10, ceci négativement.

Cependant, l'involucrine précurseur de l'enveloppe cornée et la desmogléine constituant essentiel des cornéodesmosomes sont surexprimées avec l'extrait de Myrte, notamment à la concentration de 1 pg/ml. En agissant d'une part sur les protéines du cytosquelette cellulaire (kératines K1-K10) d'une manière directe ou indirecte (au travers de l'expression de la desmogléïne) et d'autre part sur l'involucrine, précurseur essentiel de l'enveloppe des cornéocytes, l'extrait de Myrte pourrait être ainsi impliqué dans la régulation de l'expression de gènes intervenant dans la prolifération et différenciation des cellules.

L'activité de l'extrait original de Myrte sur la régulation de la prolifération des kératinocytes est. confirmée sur des cultures en monocouche.

L'étude est réalisée sur 48 heures de culture des kératinocytes humains en présence d'extrait actif de Myrte. La quantification de la population cellulaire est réalisée au Coulter Counter. L'extrait actif de Myrte présente une activité antiprolifératrice significative de 20 % à la concentration 5 µg/ml

Par ailleurs, l'extrait de Myrte selon la présente invention a également une activité anti-inflammatoire, confirmée par une étude réalisée sur un modèle cellulaire de kératinocytes humains. Une réaction inflammatoire est créée in vitro par l'addition de ionophore calcique A23187 : l'afflux de Ca++ intracellulaire déclenche la stimulation de la cascade de l'acide arachidonique et la synthèse des médiateurs inflammatoires, prostaglandines et leucotriènes. L'activité de l'extrait de Myrte est évaluée sur un des métabolites majeurs produits par le kératinocyte, la prostaglandine 6KF1 (PG 6KF1) qui est le métabolite stable de la prostacycline (PGI₂). La PG 6KF1 est mesurée par dosage immuno enzymatique (ELISA) dans les surnageants de culture. L'extrait actif de Myrte est évalué aux concentrations non cytotoxiques 0,1 µg/ml, 1 µg/ml et 10 µg/ml. Cet extrait présente une activité inhibitrice significative et dose dépendante sur la production de PG6KF1 induite par A23187 :
- 12 % à la concentration 1 pg/ml et
- 26 % à la concentration 10 µg/ml.

Cette étude démontre les propriétés anti-inflammatoires de notre extrait de Myrte.

L'extrait actif ainsi que les compositions dermatologiques et cosmétologiques en découlant, objets de la présente invention sont donc proposées dans le traitement des syndromes inflammatoires et prolifératifs cutanés et les troubles de la différentiation cutanée. D'autres indications thérapeutiques sont revendiquées comme par exemple les troubles de la kératinisation de la peau et du cuir chevelu, les parakératoses, la dermatite atopique, les xéroses, les diskératoses, les ichtyoses.

L'acné représente un désordre du follicule pilo-sébacée, particulièrement sensible au traitement par notre préparation à base de Myrte grâce à ses propriétés anti-inflammatoires et antibactériennes sur *P. acnes*. L'extrait objet de cet invention est en effet actif sur *Staphyllosoccus aureus* 6538P, *Corynebacterium xerosis* CIP 5216, *Streptosoccus mutans* ATCC 27351, *Propionibacterium acnes* ATCC 7919, *Candide albicans* ATCC 10232. La CMI, Concentration Minimale Inhibitrice sur ces différentes souches est de l'ordre de 10⁻⁶. D'où un intérêt marqué dans le traitement de l'acné et de l'assainissement de la flore cutanée.

Préparation de l'extrait actif de Myrte :

Le procédé selon la présente invention permet d'accéder à l'extrait le plus approprié en vue de traiter le psoriasis.

L'extrait selon l'invention est une fraction encore inusitée du Myrte. Cet extrait est totalement différent de l'huile essentielle de Myrte utilisée depuis des millénaires et de l'extrait polaire du brevet Pierre FABRE Dermo-cosmétique FR 2735026. Ce brevet revendique la préparation d'un extrait original de Myrte complètement différent du nôtre par sa polarité et par ses teneurs en polyphénols, flavonoïdes et tanins. Notre extrait est titré en myrtocommulone B' (environ 6%) et comprend environ 40 % en masse de triterpènes et stérols par rapport à la masse de l'extrait de Myrte. Ces composés apolaires sont absents des extraits polaires mais aussi de l'huile essentielle.

L'extrait selon la présente invention est rationnellement conçu pour agir sur les deux versants de la pathologie psoriasique (prolifération et inflammation). Les extraits polaires ainsi que l'huile essentielle ont clairement démontré leur inactivité sur la prolifération kératinocytaire.

L'indication psoriasis étant mentionnée par erreur dans GARNIER et de manière peu précise (cf référence CAZIN), l'utilisation de l'extrait selon l'invention dans le psoriasis n'est donc pas décrite dans l'art antérieur.

Une grande simplicité concernant la mise en oeuvre industrielle caractérise le procédé selon l'invention. En effet notre méthode de préparation se distingue par l'absence d'une étape obligatoire de séparation chromatographique et d'opération complexe.

Plusieurs mises en oeuvre du procédé selon l'invention peuvent être envisagées.

Selon une première variante de la mise en oeuvre du procédé selon l'invention, les parties aériennes, en fruits ou non, sèches ou non, sont extraites par un solvant aqueux ou organique de préférence de l'éthanol. L'extrait obtenu est traité par une méthode appropriée avantageusement par du charbon actif, afin d'éliminer les chlorophylles. Les composés apolaires de l'extrait "décoloré" sont isolés sélectivement soit par extraction liquide/liquide, soit avantageusement par précipitation en évaporant le solvant organique sur l'eau. Nous obtenons ainsi un extrait apolaire bien défini chimiquement que nous titrons en Myrtucommulone B'. L'extraction initiale par l'éthanol permet d'obtenir un titre en Myrtucommulone B' au produit final compris entre 4% et 8%.

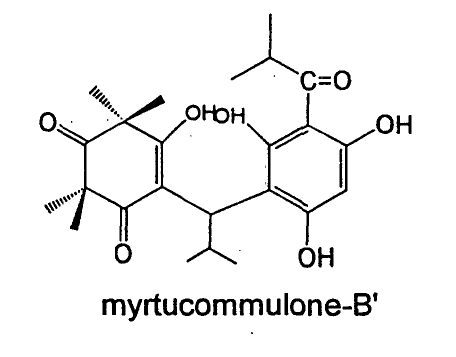

### Exemple 1

100 kg de parties aériennes broyées et sèches sont extraits à reflux pendant 1 heure par 500 litres d'ethanol (EtOH). Après filtration à froid sur toile et rinçage de la drogue épuisée par 100 litres d'EtOH, 475 litres d'extrait éthanolique ont été séparés du marc. L'extrait est traité par 800 g de charbon actif, à reflux pendant 15 minutes puis filtré à chaud sur filtre AF-15 et rincé par 25 litres d'EtOH 96° chaud. L'addition de 500 litres d'eau et l'évaporation de la totalité de l'EtOH entraîne la formation d'un précipité jaunâtre récupéré sur filtre AF-15. Ce précipité est lavé par 3 fois 30 litres d'eau. Après séchage sous vide pendant 72 heures à 30°C, broyage et tamissage nous obtenons 2,668 kg d'extrait actif de Myrte qui va entrer dans les formes galéniques présentées plus bas. Cet extrait titre 6,6 % en myrtucommulone B', 7 % en acide ursolique. Les triterpènes et stérols représentent environ 40 % en masse par rapport à la masse d'extrait de Myrte.

L'utilisation d'autres alcools comme par exemple le méthanol (MeOH), l'isopropanol ou de cétones comme par exemple l'acétone ou la méthyléthylcétone pour réaliser l'extraction initiale permettent de préparer des extraits actifs de titres compris entre 2% et 12 % en Myrtucommulone B'.

Selon une deuxième variante de la mise en oeuvre du procédé, l'extrait actif de Myrte peut également être préparé par extraction à l'aide d'un solvant tel que l'hexane, le chlorure de méthylène, l'éther isopropylique, l'acétate d'éthyle... Ce deuxième procédé n'est pas aussi avantageux que le premier car il fait intervenir des solvants autres que l'eau et l'éthanol. Il faudra donc rechercher les traces résiduelles de ces solvants organiques.

### Exemple 2

1Kg de parties aériennes est extrait à deux reprises par 5 litres d'hexane. L'extrait obtenu (9,5 litres) est concentré jusqu'à 2 litres. Après addition de 5 litres d'éthanol, le filtrat est à nouveau concentré à 2,5 litres et ajusté à 5 litres par addition d'éthanol à 96°C. L'extrait est ensuite traité à reflux pendant 15 minutes par 8 g de charbon actif. Après addition de 5 litres d'eau et évaporation totale de l'éthanol, le précipité obtenu est filtré et séché sous vide à 30°C pendant 72 heures.

On obtient les mêmes résultats en utilisant l'acétate d'éthyle, l'éther isopropylique ou le dichlorométhane pour effectuer l'extraction initiale.

L'utilisation de ces solvants pour réaliser l'extraction initiale permet de préparer des extraits actifs de titres compris entre 2 et 12% en Myrtucommulone B' selon le solvant.

Les préparations galéniques qui suivent illustrent l'invention sans en limiter la portée.

### Exemple 3 : Lotion

| | |
|---|---|
| SD-alcool 39-C | 63 ml |
| Parfum | 0,20 g |
| Copolymère acétate de vinyle / Acide crotonique | 0,15 g |
| PEG- 13 Octanoate | 0,10 g |
| Chlorure de Laurylpyridinium | 0,05g |
| Extrait de Myrte | 0,1 à 1 g |
| Eau qsp | 100 ml |

### Exemple 4 : Crème

| | |
|---|---|
| PEG-40 Sorbitan lanolate | 7,5 g |
| Paraffine | 5 g |
| Propylène glycol | 5 g |
| Alcool cétylique | 3 g |
| Polysorbate 20 | 3 g |
| Lanoline | 2 g |
| Acide salicylique | 1,5 g |
| Parfum | 0,4 g |
| Extrait de Myrte | 0,1 à 3 g |
| Eau qsp | 100 g |

### Exemple 5 : Huile

| | |
|---|---|
| Adipate de dibutyle | 15 g |
| Triglycéride caprylique / caprique | 10g |
| Extrait de Myrte | 0,1 à 5 g |
| Huile minérale qsp | 100 g |

### Exemple 6 : Savon (Syndet)

| | |
|---|---|
| Cocoglisethionate de sodium | 20 g |
| Cire d'origine minérale | 12 g |
| Alcool cétylique | 13 g |
| Amidon de blé | 20 g |
| Extrait de Myrte | 0,1 à 5 g |
| Hémisulfosuccinate de sodium qsp | 100 g |

### Exemple 7 : Mousse

| | |
|---|---|
| Gomme guar | 0,10 g |
| Huile de Jojoba | 2 g |
| P-méthoxy cinnamate d'éthyl-hexyl | 1 g |
| Vinyl pyrrolidone / DM-AE méthacrylate | 1 g |
| Copolymère PVP hexadécène | 2 g |
| Diméthyl / Triméthyl-Polysiloxane | 1 g |
| Bromure de cétrimonium | 0,40 g |
| Parfum | 0,15 g |
| Extrait de Myrte | 0,1 à 2 g |
| Eau déminéralisée qsp | 100 g |

### Exemple 8 : Solution émolliente

| | |
|---|---|
| Polysorbate 20 | 20 g |
| Esters gras de coco polyéthoxyle | 10 g |
| Alkylphénol polyéthoxyle | 10 g |
| Parfum | 1 g |
| Extrait de Myrte | 0,1 à 10 g |
| Propylène glycol qsp | 100 g |

### Exemple 9 : Huile gélifiée Corps

| | |
|---|---|
| Triglycéride Caprilyque / caprique | 20 à 30 g |
| Huile minérale | 36 g |
| Pétrolatum | 15 g |
| PEG-40 sorbitol hexaoléate | 8 g |
| Quaternium- 18 Hectorite | 5 g |
| Talloweth-60 hydrogéné Myristyl-Glycol | 5 g |
| Parfum | 0,5 g |
| Acide benzoïque | 0,3 g |
| Gallate de Propyle | 0,02 g |
| Extrait de Myrte | 0,1 à 2 g |

### Exemple 10 : Shampooing

| | |
|---|---|
| Acide salicylique pulvérisé | 1,50 g |
| Dérivé Undécylénique | 1 à 3 g |
| Polymère Quaternaire | 0,5 à 1 g |
| Alkyl sulfate éthoxylé sodique | 9 g |
| Polysorbate 20 | 5 à 7 g |
| Ethanolamide d'acides gras ou PEG 6000 distéarate | 5 g |
| Alkylamido bétaine | 1,5 g |
| Cocoamphodiacétate de sodium | 3,5 g |
| ETDA, 2Na | 0,2 g |
| Monolaurate sorbitan POE 20 | 5 g |
| Parfum | QS |
| Colorant | QS |
| Extrait de Myrte | 0,1 à 2,5 g |
| Eau purifiée qsp | 100 g |

### Exemple 11 : Shampooing

| | |
|---|---|
| Laureth sulfate de sodium | 8 g |
| Polysorbate 20 | 7 g |
| PEG- 150 distéarate | 4 g |
| Undécylénamido Mea-Sulfosuccinate de sodium | 3 g |
| Cocoamphodiacétate de sodium | 3 g |
| Acide salicylique | 1,5 g |
| Parfum | 0,5 g |
| Cocoyl-collagène hydrolysé de T.E.A. | 1 g |
| Extrait de Myrte | 0,1 à 1 g |
| Eau qsp | 100 g |

### Exemple 12 : Spray

La lotion de l'exemple 3 peut être mise sous aérosol avec un hydrocarbure comme par exemple le butane ou le propane (50% lotion et 50% propulseur) ou par un système de pompe.

Il est clair que tous ces exemples seront réalisés selon les modes de préparation bien connus des hommes de l'art, et ne sauraient en aucun cas être exhaustifs.

## Revendications

1. Extrait de parties aériennes de Myrte **caractérisé en ce que** cet extrait est une fraction apolaire comprenant des myrtucommulones, des triterpènes et des stérols, le titre de myrtucommulone B' étant compris entre 2% et 12 % et le titre du mélange "triterpènes et stérols" étant compris entre 30 % et 50 % et tel que cet extrait est essentiellement exempt de chlorophylles.

2. Extrait selon la revendication 1 tel que le titre de myrtucommulone B' est compris entre 4% et 8%.

3. Extrait selon la revendication 1 ou 2 tel que le titre de myrtucommulone B' est compris entre 5% et 7% et le titre du mélange "triterpènes et stérols" est compris entre 37 % et 43 %.

4. Procédé de préparation d'un extrait de parties aériennes de Myrte selon l'une des revendications précédentes **caractérisé en ce qu'**il comprend une étape dans laquelle les parties aériennes de Myrte sont soumises à une extraction au moyen d'un solvant, une étape d'élimination des chlorophylles et une étape dans laquelle les composés apolaires sont isolés.

5. Procédé selon la revendication 4 tel que le solvant organique utilisé pour effectuer une extraction sur les parties aériennes de la plante est un alcool ou une cétone.

6. Procédé selon la revendication 4 ou 5 tel que le solvant organique utilisé pour effectuer une extraction sur les parties aériennes de la plante est choisi dans le groupe formé par l'éthanol, le méthanol, l'isopropanol, l'acétone, la méthyléthylacétone, l'hexane, le chlorure de méthylène, l'éther isopropylique, l'acétate d'éthyle.

7. Procédé selon l'une des revendications 3 à 6, tel que l'élimination des chlorophylles est effectuée au moyen de charbon actif.

8. Procédé selon l'une des revendications 3 à 7, tel que les composés apolaires sont isolés par au moins un des procédés suivants, une extraction liquide/liquide, une précipitation en évaporant le solvant organique sur l'eau.

9. Composition dermatologique et/ou cosmétique telle qu'elle comprend un extrait selon l'une des revendications 1, 2, ou 3.

10. Composition selon la revendication 9 pour le traitement du psoriasis.

11. Composition selon la revendication 9 pour le traitement des troubles de la kératinisation de la peau et du cuir chevelu, les parakératoses, la dermatite atopique, les xéroses, les diskératoses, les ichtyoses, l'acné.

## Claims

1. Extract of aerial parts of myrtle, **characterized in that** this extract is a nonpolar fraction comprising myrtucommulones, triterpenes and sterols, the content of myrtucommulone B' being between 2% and 12% and the content of the "triterpenes and sterols" mixture being between 30% and 50%, and such that this extract is essentially devoid of chlorophylls.

2. Extract according to Claim 1, such that the content of myrtucommulone B' is between 4% and 8%.

3. Extract according to Claim 1 or 2, such that the content of myrtucommulone B' is between 5% and 7% and the content of the "triterpenes and sterols" mixture is between 37% and 43%.

4. Process for the preparation of an extract of aerial parts of myrtle according to one of the preceding claims, **characterized in that** it comprises a stage in which the aerial parts of myrtle are subjected to an extraction by means of a solvent, a stage of removal of the chlorophylls and a stage in which the nonpolar compounds are isolated.

5. Process according to Claim 4, such that the organic solvent used to carry out an extraction on the aerial parts of the plant is an alcohol or a ketone.

6. Process according to Claim 4 or 5, such that the organic solvent used to carry out an extraction on the aerial parts of the plant is chosen from the group formed by ethanol, methanol, isopropanol, acetone, methyl ethyl ketone, hexane, methylene chloride, isopropyl ether and ethyl acetate.

7. Process according to one of Claims 3 to 6, such that the removal of the chlorophylls is carried out by means of active charcoal.

8. Process according to one of Claims 3 to 7, such that the nonpolar compounds are isolated by at least one of the following processes: liquid/liquid extraction or precipitation by evaporating the organic solvent with respect to the water.

9. Dermatological and/or cosmetic composition, such that it comprises an extract according to one of Claims 1, 2 and 3.

10. Composition according to Claim 9, for the treatment of psoriasis.

11. Composition according to Claim 9, for the treatment of disorders of keratinization of the skin and scalp, parakeratoses, atopic dermatitis, xeroses, dyskeratoses, ichthyoses or acne.

## Patentansprüche

1. Extrakt von Luftteilen von Myrthe, **dadurch gekennzeichnet, dass** dieser Extrakt eine unpolare Fraktion ist, die Myrtucommulone, Triterpene und Sterole umfasst, wobei der Myrtucommulon-Titer B' 2 % bis 12 % beträgt und der Titer der Mischung "Triterpene und Sterole" 30 % bis 50 % beträgt, und dass dieser Extrakt im Wesentlichen frei von Chlorophyllen ist,

2. Extrakt nach Anspruch 1, In dem der Myrtucommulon-Titer B' 4 % bis 8 % beträgt.

3. Extrakt nach Anspruch 1 oder 2, in dem der Myrtucommulon-Titer B' 5 % bis 7 % beträgt und der Titer der Mischung "Triterpene und Sterole" 37 % bis 43 % beträgt.

4. Verfahren zur Herstellung eines Extraktes von Luftteilen von Myrthe gemäß einem der vorangehenden Ansprüche, **dadurch gekennzeichnet, dass** es einen Schritt, in dem die Luftteile von Myrthe einer Extraktion mittels eines Lösungsmittels unterzogen werden, einen Schritt der Entfernung von Chlorophyllen und einen Schritt, in welchem die unpolaren Verbindungen isollert werden, umfasst.

5. Verfahren nach Anspruch 4, in dem das organische Lösungsmittel, das verwendet wird, um eine Extraktion der Luftteile der Pflanze zu bewirken, ein Alkohol oder ein Keton ist.

6. Verfahren nach Anspruch 4 oder 5, in dem das organische Lösungsmittel, das verwendet wird, um eine Extraktion der Luftteile der Pflanze zu bewirken, aus der Gruppe ausgewählt ist, die durch Ethanol, Methanol, Isopropanol, Aceton, Methylethylketon, Hexan, Methylenchlorid, Isopropylether, Ethylacetat gebildet wird.

7. Verfahren nach einem der Ansprüche 3 bis 6, in dem die Entfernung der Chlorophylle mittels Aktivkohle bewirkt wird.

8. Verfahren nach einem der Ansprüche 3 bis 7, in dem die unpolaren Verbindungen mittels mindestens eines der folgenden Verfahren isoliert werden: eine Flüssig/Flüssig-Extraktion, eine Ausfällung, indem man das organische Lösungsmittel über Wasser verdampft.

9. Dermatologische und/oder kosmetische Zusammensetzung, die einen Extrakt gemäß einem der Ansprüche 1, 2 oder 3 umfasst.

10. Zusammensetzung nach Anspruch 9 für die Behandlung von Psoriasis.

11. Zusammensetzung nach Anspruch 9 für die Behandlung von Störungen der Keratinisierung der Haut und der Kopfhaut, von Parakeratosen, atopischer Dermatitis, Xerosen, Diskeratosen, Ichtyosen, Akne.
